Europäisches Patentamt

**European Patent Office**

**Office européen des brevets**

(19)

(11) Publication number: **0 196 143**
A2

# EUROPEAN PATENT APPLICATION

(12)

(21) Application number: **86200498.3**

(22) Date of filing: **24.03.86**

(51) Int. Cl.⁴: **A 61 L 27/00,** A 61 L 25/00,
A 61 K 6/02

(30) Priority: **25.03.85 NL 8500866**

(43) Date of publication of application: **01.10.86**
**Bulletin 86/40**

(84) Designated Contracting States: **AT BE CH DE FR GB IT LI**
**LU NL SE**

(71) Applicant: **Stichting Biomaterials Science Center, VU,**
**"BSC-VU", De Boelelaan 1115, NL-1081 HV Amsterdam**
**(NL)**

(72) Inventor: **de Groot, Klaas, L. van Wijkplein 6, NL-2101 EL**
**Heemstede (NL)**

(74) Representative: **Smulders, Theodorus A.H.J. et al,**
**Vereenigde Octrooibureaux Nieuwe Parklaan 107,**
**NL-2587 BP 's-Gravenhage (NL)**

(54) **A method of preparing an implant material, and two-component kit suitable therefor.**

(57) The invention relates to a method of preparing an implant material in which particles of a bioactive ceramic biomaterial, such as apatite, of a size of at least 200 μm are mixed with water and a water-soluble hydrocolloid. The invention also relates to a two-component kit for preparing the implant material.

EP 0 196 143 A2

Title:
A method of preparing an implant material, and two-
component kit suitable therefor.

This invention relates to a method of preparing
an implant material on the basis of particulate bioactive
ceramic biomaterial.

In an article by K. Groot in Med. Progr. Technol.
9, 129-136 (1982), implant materials are disclosed,
including particulate bioactive ceramic biomaterials,
that can be used for filling holes in bone and tooth
tissue. The term "biomaterials" refers to materials
that can be used to replace bone and tooth tissue. According
to their physico-chemical properties, biomaterials can
be distinguished into (1) metals, (2) plastics, (3)
ceramic materials, and (4) composite materials, for
example, metal-ceramic combinations (cermets). Biologically,
these products can be distinguished into (1) inert materials
and (2) bioactive materials, according to whether a
direct bond is or is not formed between the bone tissue,
connective tissue, or epithelium tissue and the implanted
material (the implant).

Biologically, ceramic implants are generally
regarded as the most acceptable, sometimes termed the
most biocompatible. Examples of inert ceramic biomaterials
are aluminum oxide and a diamond-like carbon derivative.
Bioactive ceramic biomaterials are materials which at
least in vivo and at least on the outside exhibit a
physico-chemical structure and composition which correspond
to one or more structures and compositions which occur
in the inorganic phase of bone or tooth tissue. Examples
of such bioactive ceramic biomaterials are apatite,
fluoroapatite, $\alpha$- or $\beta$-whitlockite, calcium carbonate,
magnesium containing $\beta$-whitlockite, calcium phosphate,
and glass-ceramic such as the commercial products Ceravital (Leitz, Germany) and Bioglas.

These known bioactive ceramic biomaterials are presented as implant materials in the particulate state. They may, for example, be introduced into the hole to be filled in the bone or tooth tissue by means of a needleless syringe together with water or saline.

One disadvantage of this known implant material, however, is that both during application and afterwards the particles are difficult to maintain in position. A significant part of the particles flows away, finds its way into wrong places, and may later cause problems there. Indeed, the handling of the known implant material is difficult, and particular measures have to be taken for the particles to be kept in position after application, such as the use of a cover over the bone or tooth tissue defect treated. Moreover, only relatively small spaces can be filled with these implant materials.

The present invention provides a method of the above kind which eliminates these disadvantages and is characterized in that particles of bioactive ceramic biomaterial with a particle size of at least 200 $\mu$m are made up into a mixture by means of water in the presence of a water-soluble hydrocolloid in the particulate material and/or in the water. The hydrocolloid is characterized in that an aqueous solution when cooled to a temperature slightly above body temperature (38-40$^{\circ}$C) exhibits a sol-gel transition. The gel-sol transition generally takes place not at the same temperature, but a higher temperature (for example, 75-80$^{\circ}$C).

The method according to the invention comprises heating the mixture to above the gel-sol transition temperature, whereafter the resulting sol is cooled to slightly above the sol-gel transition temperature. Immediately after the implantation of the powder-liquid mixture the gel state is obtained by cooling to body temperature, and so its consistency is hard. These physical reactions have been used before in preparing so-called hydrocolloid (or agar-agar) casts, that is to say, materials with which accurate negatives can be obtained from teeth (see Peyton et al., Restorative Dental Materials, 4th. Edition, C.V. Mosby Co., U.S.A., (1971), pp 187191, and Greener et al., Materials Science in Dentistry, Williams and Wilkins Co., Baltimore, U.S.A. (1972), pp 309-314).

Known materials for making casts commonly also contain fillers to produce good processing consistency. A typical filler is, for example, calcium carbonate. In order to permit making accurate casts of fine detail, however, very fine filler is used, in particle sizes of no more than 20 to 30 μm, preferably no more than 5 to 10 μm. As regards the composition of the implant material according to the present invention, this is distinguished from known materials for making casts in the presence of particles having sizes of at least 200 μm.

According to the invention, it is preferable to use particles of bioactive ceramic biomaterial having a particle size of at least 250 μm. A larger particle size implies larger interspaces between the individual particles, which interspaces are sometimes referred to as macropores (see K. de Groot in Ceramics in Surgery, Ed. P. Vincenzini, 1983, Elsevier Scientific Publishing Company, Amsterdam, pp 79-80), and promotes the ingrowth of bone and tooth tissue. Particles which are too small, i.e. less than 200 μm, would impede bone ingrowth, and are therefore unsuitable for use in implant material. A practical upper limit for the particle size is about 4 mm, although larger particles are suitable.

As regards the composition of the implant material according to the invention, it is preferable that 80-99 % by weight of the dry matter is constituted by particles of bioactive ceramic biomaterial, and 1-20% by water-soluble hydrocolloid.

In order to produce a paste-like consistency that is easy to process, it is preferable that 30-50 ml water is used to 100 g of dry matter. Lower or higher proportions, for example 20 ml or 60 ml water to 100 g dry matter, however, are good to use.

In principle, the water-soluble hydrocolloid may be present in the particulate component, while the aqueous liquid consists of water.

The other possibility, i.e., that the hydrocolloid is present in the water, is also comprised by the invention.

One important advantage of the implant material according to the invention is that it is much simpler to process than the known particulate material. After mixing the components, a kneadable paste is obtained, which without any loss of material can be effectively applied to the location in question and moulded. The particles of bioactive ceramic biomaterial present in the paste cannot flow away, either during application, or later. The hardened hydrocolloid is biocompatible and, in time, is absorbed in vivo. At the same time, the function of the hydrocolloid is taken over by ingrown bone or connective tissue, so that a perfect filling of the bone or tooth tissue defect results. The paste-like implant material according to the invention is in addition suitable for filling larger holes and cavities, in particular between bone and a prosthesis introduced.

The invention also provides a two-component kit for preparing an implant material, comprising a particulate component and a aqueous liquid with the particulate component and/or the aqueous liquid containing a water-soluble hydrocolloid.

As observed before with reference to the method according to the invention, it is preferable for the particulate component to contain particles of bioactive ceramic biomaterial having a particle size of at least 250 $\mu$m; that 80-99 % by weight of the dry matter is constituted by particles of bioactive ceramic biomaterial, 1-20 % by weight by water-soluble hydrocolloid; that the water-soluble hydrocolloid is present in the particulate component; and that 30-50 ml water is present to 100 g of dry matter.

The invention is illustrated in and by the following examples.

Example I

A particulate material was prepared, consisting of 3 g of hydrocolloid and 90 g apatite particles having a particle size of 250-350 μm. This particulate material was mixed by shaking with 400 ml water, which resulted in a readily processable paste.

Example II

A powdered material was prepared, consisting of 3 g hydrocolloid and 90 g calcium carbonate particles having a particle size of 250-350 μm. This particulate material was mixed by shaking with 40 g water, whereby a readily processable paste was obtained.

Example III

A particulate material was prepared, consisting of 4 g hydrocolloid and 70 g β-whitlockite particles having a particle size of 2-4 mm. This particulate material was mixed by shaking with 40 ml water, whereby a readily processable paste was obtained.

C L A I M S

1.      A method of preparing an implant material
on the basis of particulate, bioactive ceramic biomaterial,
characterized in that particles of bioactive ceramic
biomaterial with a particle size of at least 200 μm
are made up into a mixture by means of water in the
presence of a water-soluble hydrocolloid in the particulate
material and/or in the water.

2.      A method as claimed in claim 1, characterized
by using particles of bioactive ceramic biomaterial
having a particle size of at least 250 μm.

3.      A method as claimed in claim 1 or 2, characterized
in that 80-99 % by weight of the dry content is formed
by particles of bioactive ceramic material and 1-20
% by weight by water-soluble hydrocolloid.

4.      A method as claimed in any of claims 1-3,
characterized in that the water-soluble hydrocolloid
is supplied via the particulate material.

5.      A method as claimed in any of claims 1-4,
characterized in that 30-50 ml water to 100 g of dry
matter is used.

6.      A two-component kit for preparing an implant
material comprising a particulate component and an aqueous
liquid with the particulate component and/or the aqueous
liquid containing a water-soluble hydrocolloid.

7.      A two-component kit as claimed in claim 6,
characterized in that the particulate component contains
particles of bioactive ceramic biomaterial with a particle
size of at least 250 μm.

8.      A two-component kit as claimed in claim 6
or 7, characterized in that 80-99 % by weight of the
dry matter is constituted by particles of bioactive
ceramic biomaterial, and 1-20 % by weight by water-soluble hydrocolloid.

9.      A two-component kit as claimed in any of claims
6-8, characterized in that the water-soluble hydrocolloid
is present in the particulate component.

10.      A two-component kit as claimed in any of claims
6-9, characterized in that 30-50 ml water is present
to 100 g of dry matter.